# EUROPEAN PATENT SPECIFICATION

(11) **EP 3 847 984 B1**
(45) Date of publication and mention of the grant of the patent: **18.12.2024**
(21) Application number: 21156920.7
(22) Date of filing: 01.06.2018
(51) Int. Cl.: A61B 18/02, A61B 18/18, A61B 18/00

(54) **ELECTROSURGICAL INSTRUMENT FOR FREEZING AND ABLATING BIOLOGICAL TISSUE**
ELEKTROCHIRURGISCHES INSTRUMENT ZUM GEFRIEREN UND ZUR ABLATION VON BIOLOGISCHEM GEWEBE
INSTRUMENT ÉLECTROCHIRURGICAL POUR LA CONGÉLATION ET L'ABLATION DE TISSU BIOLOGIQUE

(30) Priority: 01.06.2017 GB 201708725
(43) Date of publication of application: 14.07.2021
(62) Divisional of application: 18730674.1
(73) Proprietor: Creo Medical Limited, Chepstow, Wales NP16 5UH (GB)
(72) Inventor: HANCOCK, Christopher Paul, Bath, Gwent BA1 4LN (GB); WHITE, Malcolm, Chepstow, Gwent NP16 5UH (GB); BURN, Patrick, Chepstow, Gwent NP16 5UH (GB); CLEGG, Peter, Chepstow, Gwent NP16 5UH (GB); SHAH, Pallav, London, Greater London N21 2EP (GB)
(74) Representative: Mewburn Ellis LLP

(56) References cited:
- EP-A1- 3 618 745
- EP-B1- 3 618 745
- WO-A1-2010/048335
- GB-A- 2 487 199
- GB-A- 2 503 673
- US-A1- 2013 289 678
- US-A1- 2014 276 743
- US-B2- 9 226 787

## Description

### FIELD OF THE INVENTION

The invention relates to an electrosurgical apparatus for ablating biological tissue using microwave energy. In particular, the electrosurgical apparatus includes a probe can be used in the lungs or in the uterus, for example to ablate tumours, lesions or fibroids and to treat asthma. The probe may be inserted through a working channel of a surgical scoping device or catheter, or may be used in laparoscopic surgery or open surgery.

### BACKGROUND TO THE INVENTION

It is inherently difficult to gain access to lung tumours due to the small dimensions of the bronchial tree, especially towards the peripheral regions where small nodules are likely to develop. This has resulted in many treatment options being employed such as chemotherapy (targeted medicine, anti-cancer drugs (chemotherapeutic agents)), radiotherapy (delivery of ionizing radiation), surgery (invasive and minimally invasive) and RF/microwave ablation. Surgical procedures for the removal of lung tumours include pneumonectomy (removal of one lung), lobectomy (removal of a lobe), sleeve lobectomy (resection of a lobe along with part of the bronchus that attaches to it), wedge resection (removal of a wedge shaped portion of lung) and segmentectomy/segment resection (resection of a specific lung segment).

Biological tissue is largely composed of water. Human soft organ tissue is typically between 70% and 80% water content. Water molecules have a permanent electric dipole moment, meaning that a charge imbalance exists across the molecule. This charge imbalance causes the molecules to move in response to the forces generated by application of a time varying electric field as the molecules rotate to align their electric dipole moment with the polarity of the applied field. At microwave frequencies, rapid molecular oscillations result in frictional heating and consequential dissipation of the field energy in the form of heat. This is known as dielectric heating.

This principle is harnessed in microwave ablation therapies, where water molecules in target tissue are rapidly heated by application of a localised electromagnetic field at microwave frequencies, resulting in tissue coagulation and cell death. It is known to use microwave emitting probes to treat various conditions in the lungs and other body tissues. For example, in the lungs, microwave radiation can be used to treat asthma and ablate tumours or lesions.

Conventional microwave ablation probes are designed to be inserted into the patient percutaneously. However, such probes are difficult to locate percutaneously into a moving lung, which can lead to complications such as pneumothorax and haemothorax (air and blood within the pleural cavity respectively). Other microwave ablation probes can be delivered to a target site by a surgical scoping device (e.g. a bronchoscope or other type of endoscope) which can be run through channels in the body such as airways. This allows for minimally invasive treatments, which can reduce the mortality rate of patients and reduce intraoperative and postoperative complication rates.

Using a probe to deliver the microwave energy to target tissue is desirable because the radiating portion can be positioned close to the target site, such that a high proportion of power can be transmitted to the target site and a lower proportion is lost to the surrounding healthy tissue. This reduces side effects of treatment as well as increasing efficiency.

Because microwave energy rapidly dissipates in biological tissues, biological tissues are often described as lossy materials. Microwave energy radiated from an ablation probe therefore does not propagate far in biological tissue before it is completely dissipated. The volume over which microwave energy is dissipated in biological tissue is frequency dependent and can be described using a quantity called skin depth. Skin depth is defined as the distance away from the surface a radiating antenna of the ablation probe at which microwave power has been reduced by a factor of 1/e compared to the total power radiated by the antenna (where e is the number whose natural logarithm is equal to one).

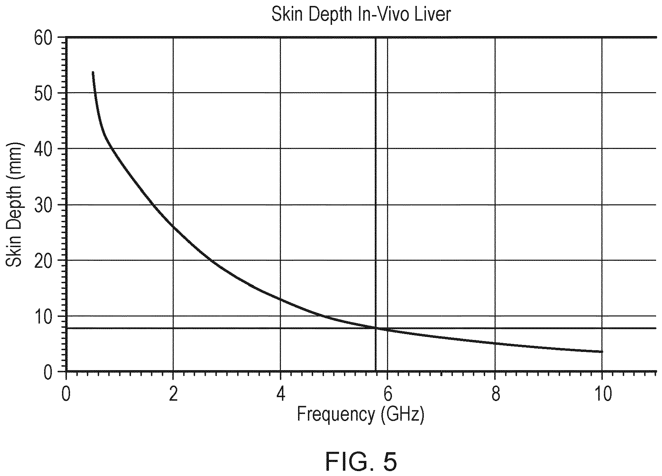

By way of example, Fig. 5 shows a graph of skin depth versus frequency over a frequency range of 0.5 - 10 GHz, which covers a typical range of microwave ablation frequencies. The skin depth was calculated for in-vivo liver, using measured complex permittivity data. As shown in Fig. 5, at an example ablation frequency of 5.8 GHz the skin depth is around 8 mm. This means that most of the microwave energy is dissipated less than 1 cm away from the surface of the radiating antenna. The size of a treatment area for microwave ablation probes is therefore limited to a small region around the radiating antenna.

The size of the treatment area can be increased by increasing the amount of microwave energy delivered to the radiating antenna, i.e. the power transferred to the antenna. However, the cable that conveys the energy to the antenna is itself lossy, and typically the rate of loss increases as the diameter of the cable decreases. This effectively limits the amount of energy which can be delivered, in order to avoid collateral damage caused by cable heating. Increasing the amount of microwave energy can also cause the probe to generate large amounts of heat, such that it is necessary to use a cooling mechanism to avoid damage to the probe and/or patient.

GB 2 487 199 A discloses an electrosurgical device with fluid conduit. GB 2 503 673 A refers to an electrosurgical device with convex under surface. WO 2010/048335 A1 discloses methods and devices for applying energy to bodily tissues. US 9 226 787 B2 refers to a catheter having temperature controlled anchor and related methods. US 2014/276743 A1 discloses methods and devices for fluid enhanced microwave ablation therapy. US2013289678 discloses a device combining RF heating with cryogenic freezing of tissue and the detection of freezing of tissue to direct the hyperthermic energy to the tissue.

### SUMMARY OF THE INVENTION

The invention is defined by the claims.

At its most general, the present invention provides an electrosurgical apparatus for applying microwave energy to biological tissue, where the apparatus is capable of freezing biological tissue in a region around a radiating tip portion of the apparatus and applying microwave energy to the frozen tissue. As water molecules in frozen tissue have reduced vibrational and rotational degrees of freedom compared to non-frozen tissue, less energy is lost to dielectric heating when microwave energy is transmitted through frozen tissue. Thus, by freezing the region around the radiating tip portion, microwave energy radiated from the radiating tip portion can be transmitted through the frozen region with low losses and into tissue surrounding the frozen region. This enables the size of the treatment area to be increased compared with conventional microwave ablation probes, without having to increase the amount of microwave energy delivered to the radiating tip portion. Once the tissue surrounding the frozen region has been ablated with microwave energy, the frozen region can be allowed to progressively thaw so that it will dissipate microwave energy and be ablated. The apparatus of the invention also enables various combinations of microwave energy and tissue freezing to be used to effectively ablate biological tissue. The electrosurgical device can be configured to be fed through the working channel of an endoscope, so that it can be used to carry out minimally invasive surgical procedures.

There is provided an electrosurgical instrument for treating biological tissue, the instrument comprising: a transmission line for conveying microwave electromagnetic (EM) energy; a radiating tip mounted at a distal end of the transmission line to receive and radiate the microwave EM energy from the transmission line into a treatment zone around the radiating tip; a fluid feed for conveying a tissue-freezing fluid to the treatment zone; and a thermal transfer portion connected to receive the tissue-freezing fluid from the fluid feed at a distal end of the transmission line, wherein the thermal transfer portion is arranged to provide thermal communication between the tissue-freezing fluid and biological tissue in the treatment zone to freeze the biological tissue in the treatment zone.

The radiating tip may comprise a microwave antenna. The antenna may be a conventional monopole antenna formed on the end of the transmission line. The transmission line may be a coaxial transmission line, e.g. a conventional coaxial cable. An inner conductor of the coaxial cable may be connected to a radiating tip of the microwave antenna from which microwave energy can radiate. The radiating tip may include one or more dielectric materials to provide dielectric loading of the antenna, in order to enhance or shape the energy emission profile of the microwave antenna. The coaxial feed cable includes an outer conductor which is separated from the inner conductor by a dielectric material.

The electrosurgical instrument can be used to apply microwave energy to matter in its vicinity, such as biological tissue, fluids or other materials. Microwave energy can cause dielectric heating in biological tissue, which can be used to ablate tissue in a localised volume around the antenna. Therefore, by inserting the antenna directly into a treatment zone, including e.g. a tumour, lesion or fibroid, microwave energy can be applied to tissue in the treatment zone in order to ablate it.

The electrosurgical instrument enables biological tissue in the treatment zone located around the radiating tip to be frozen. Herein, biological tissue is said to be "frozen" if the water contained in the biological tissue is in ice form, i.e. water molecules in the biological tissue are held in a crystal structure. Tissue is said to be "non-frozen" if the water molecules in the tissue are in a liquid state. Frozen tissue has a lower dielectric loss factor at microwave frequencies compared to non-frozen tissue, thus enabling it to transmit microwave energy more efficiently than non-frozen tissue. The dielectric loss factor is related to the imaginary part of a material's permittivity, and is indicative of energy dissipation in the material.

The tissue-freezing fluid may be a cryogenic liquid or gas, and may be referred to herein as a cryogen. The term "cryogen" may refers to a substance which is used to produce temperatures below 0°C. Liquid, gas or solid cryogens may be used. Suitable cryogens include, but are not limited to liquid nitrogen, liquid carbon dioxide and liquid nitrous oxide. The fluid feed may be provided with a thermal insulation layer made of a thermally insulating material and/or a vacuum jacket to prevent other parts of the apparatus from being cooled by the cryogen. This can also ensure that only tissue in the treatment zone is frozen, and that other parts of the patient which may be in close proximity to the cryogen conveying conduit are not affected by the cryogen.

The flow of tissue-freezing fluid through the fluid feed may be adjusted to control the cooling power of the electrosurgical instrument. For example. the flow of tissue-freezing fluid may be increased to increase the cooling power, thereby causing tissue in the treatment zone to freeze. The flow of tissue-freezing fluid may be reduced or stopped to allow tissue in the treatment zone to thaw. The cooling power may determine the volume of tissue which is frozen (e.g. the greater the cooling power, the larger the volume of tissue which is frozen). Herein the term "cooling power" is used to describe the instrument's ability to remove heat from an area.

The thermal transfer portion may be arranged so that it is cooled by the tissue-freezing fluid delivered by the fluid feed and so that it may come in direct contact with the biological tissue in the treatment zone. In this manner, the thermal transfer portion can be brought into contact with tissue which is to be frozen. The tissue-freezing fluid can then cool the thermal transfer portion, which in turn freezes the tissue. The thermal transfer portion may be made of a thermally conductive material such as a metal or other suitable material. The thermal transfer portion may have a first portion which is configured to come into contact with the tissue-freezing fluid from the fluid feed and a second portion which is configured to come into contact with biological tissue. A heater may be mounted on or near the thermal transfer portion in order to enable more accurate temperature control of the thermal transfer portion.

The fluid feed may be arranged to circulate the tissue-freezing fluid through the thermal transfer portion. For example, the fluid feed may comprise a delivery conduit for conveying the tissue-freezing fluid to the thermal transfer portion, and an exhaust conduit for conveying the tissue-freezing fluid away from the thermal transfer portion. This prevents build-up of pressure in the cryogenic instrument. The term "used cryogen" refers to cryogen which has come into contact with the first portion of the tissue-freezing element, and thereby absorbed heat from the tissue-freezing element.

The thermal transfer portion may comprise a enclosed reservoir for receiving the tissue-freezing fluid, e.g. in the first portion thereof. An inlet of the reservoir may be connected to an outlet of the delivery conduit, and an outlet of the reservoir may be connected to an inlet of the exhaust conduit. The instrument may comprise a pump for causing the tissue-freezing fluid flow through the instrument.

The second portion of the thermal transfer portion may include a protective outer layer made of a biologically inert material.

In some examples, the instrument circulates the tissue-freezing fluid in a closed circuit. In other examples, the thermal transfer portion may include an outlet for delivering the tissue-freezing fluid into the treatment zone. The outlet may include a nozzle arranged to spray the tissue-freezing fluid into the treatment zone. In such an example, the instrument may further include a decompression tube through which gas in the treatment zone may escape. This avoids build-up of pressure in the treatment zone, which could cause internal damage to the patient. This is particularly important where a liquid cryogen is used, as the liquid cryogen will rapidly expand into a gas when it comes into contact with warm tissue.

The decompression tube may have a gas inlet located near the distal end of instrument, through which gas in the treatment zone may enter, and an exhaust outlet located near a proximal end of the instrument through which the gas may exit. The gas inlet and/or exhaust outlet may be fitted with one way valves to prevent gas entering into the treatment zone through the decompression tube. The gas inlet and/or exhaust outlet may be fitted with a pressure relief valve configured to automatically open when pressure in the treatment zone reaches a predetermined threshold, to ensure that pressure in the treatment zone is kept at a safe level. The instrument may also include a pressure sensor located near its distal end for monitoring pressure in the treatment zone.

The thermal transfer portion may have other configurations. For example, the thermal transfer portion may include a balloon which is fluidly connected to the fluid feed so that it can be inflated with the tissue-freezing fluid.

The thermal transfer portion may include a tissue-freezing element that is movable between an exposed position where it protrudes distally beyond the radiating tip, and a retracted position in which it is set back from the radiating tip. The tissue-freezing element may be moved between the two positions using one or more control wires. This enables the tissue-freezing element to be deployed only when the user wishes to make use of the freezing functionality, so that the tissue-freezing element does not cause any accidental injuries when not in use. The distal end of the electrosurgical instrument may also include a sheath or protective hull which covers the tissue-freezing element when it is in the retracted position, to further improve safety.

The transmission line and the fluid feed may be within a common cable. In some examples, the fluid feed is integrated with the transmission line. For example, the transmission line may be a coaxial transmission line comprising an inner conductor, an outer conductor, and a dielectric material separating the inner conductor and the outer conductor, and wherein the inner conductor is hollow to provide a passageway for the fluid feed. The tissue-freezing element may be slidably mounted in the passageway. An inner wall of the hollow inner conductor may form part of the cryogen delivery conduit. The cryogen may therefore also serve to cool the coaxial feed cable.

The integration of the two functionalities can provide a compact device and simplify ablation procedures, as it does not require different components to be inserted or removed from the working channel of an endoscope during an ablation procedure.

The instrument may include a temperature sensor mounted at a distal end of the transmission line to detect a temperature of the treatment zone. Control of the tissue-freezing fluid flow and microwave energy delivery may be based on a detected temperature.

The instrument may be used in an electrosurgical apparatus for treating biological tissue, the apparatus also comprising an electrosurgical generator arranged to supply microwave electromagnetic (EM) energy, and a tissue-freezing fluid supply. The electrosurgical instrument may be connected to receive the microwave EM energy from the electrosurgical generator and to receive the tissue-freezing fluid from the tissue-freezing fluid supply. The apparatus may further comprise a controller configured to: cause the tissue-freezing fluid to flow through the fluid feed to the thermal transfer portion to freeze biological tissue in the treatment zone; detect conditions in the treatment zone to determine if biological tissue in the treatment zone is frozen; and in response to determining that biological tissue in the treatment zone is frozen, cause the microwave energy to be delivered from the radiating tip. The controller may also be configured to, in response to determining that biological tissue in the treatment zone is frozen, reduce or stop the flow of tissue-freezing fluid through the fluid feed. The controller may be configured to determine whether the biological tissue in the treatment zone is frozen based on any one or more of: a detected impedance of the treatment zone, and a detected temperature of the treatment zone.

The controller may be a conventional computing device which is operatively connected to the instrument to control the flow of tissue-freezing fluid. For example the controller may control one or more valves and/or a pump which can be used to regulate the flow of cryogen through the delivery conduit. Reducing or stopping the flow of cryogen when the controller determines that the biological tissue in the treatment zone is frozen avoids the risk of freezing tissue outside of a desired target area, which could cause damage to surrounding healthy tissue.

Conversely, if the controller determines that the biological tissue in the treatment zone is not frozen, or that it is thawing, the controller may increase the flow of cryogen through the delivery conduit in order to freeze the tissue in the treatment zone. The controller may also be configured to regularly monitor the state (e.g. frozen or non-frozen) of tissue in the treatment zone and adjust the flow of cryogen through the cryogen conveying conduit so that the tissue in the treatment zone reaches a desired state (e.g. frozen or non-frozen). The controller therefore provides an automated mechanism for controlling the freezing of tissue in the treatment zone, and reduces the risk of damaging tissue outside the treatment zone.

In some embodiments, the controller may be configured to, in response to determining that the biological tissue in the treatment zone is frozen, cause the electrosurgical instrument to deliver microwave energy to the biological tissue in the treatment zone. The controller may be configured to control the generator which is connected to provide the microwave energy to the electrosurgical instrument. In some examples, the controller may be integrated with the generator. As the biological tissue in the treatment zone is frozen when the microwave energy is delivered, the microwave energy may be transmitted with low losses by the frozen tissue to surrounding non-frozen tissue which can be ablated by the microwave energy. This can increase the effective volume of tissue treated.

The controller may use several methods to determine whether the biological tissue in the treatment zone is frozen. In some embodiments, the electrosurgical apparatus may further include a sensor disposed near the radiating tip portion, and the controller may be configured to determine whether the biological tissue in the treatment zone is frozen based on a measurement obtained from the sensor. The sensor may be any suitable sensor for measuring a property of biological tissue, where the property varies according the tissue's state (i.e. frozen or non-frozen). For example, the sensor may be a temperature sensor arranged to measure the temperature of biological tissue in the treatment zone. The sensor may be a pressure sensor arranged to detect a change in pressure when the tissue freezes (e.g. because of the expansion of water when it freezes).

In other examples, the controller may be configured to determine whether the biological tissue in the treatment zone is frozen based on an impedance measurement of the biological tissue in the treatment zone. An impedance measurement may be carried out for example by delivering a pulse of microwave energy to the radiating tip portion, and measuring microwave energy which is reflected back up the coaxial feed cable. Microwave energy may be reflected back at the radiating tip portion due to an impedance mismatch between the radiating tip portion and the biological tissue in the treatment zone. The impedance of biological tissue is a function of the permittivity and the conductivity of the biological tissue at a frequency of interest, and hence depends on whether the biological tissue is frozen or non-frozen. By measuring the reflected microwave energy, the impedance of the biological tissue in the treatment zone can be estimated in order to determine whether the biological tissue is frozen or not. A low power microwave pulse can be used to measure the impedance of the biological tissue, so that the measurement does not cause any tissue ablation.

Optionally, the electrosurgical apparatus may also include a separate fluid delivery mechanism for transporting fluid to and from the treatment zone. The fluid delivery mechanism may be used to inject a fluid into the treatment zone and/or aspirate fluid from the treatment zone. The fluid delivery mechanism may include a flexible fluid conveying conduit that extends along the coaxial cable, and a rigid insertion element in communication with a distal end of the fluid conveying conduit and arranged to extend into the treatment zone. For example the rigid insertion element may be a hollow needle which can be exposed using one or more control wires in order to pierce tissue in the treatment zone. The fluid delivery mechanism may also be used to aspirate tissue samples from the treatment zone in order to perform a biopsy.

The electrosurgical apparatus discussed above may form part of a complete electrosurgical system. For example, the apparatus may include a surgical scoping device having an flexible insertion cord for non-percutaneous insertion into a patient's body, wherein the flexible insertion cord has an instrument channel running along its length, and wherein the electrosurgical instrument is dimensioned to fit within the instrument channel.

It should be noted that the microwave ablation and tissue-freezing functionalities of the instrument may be used independently. For example, microwave energy may be applied directly to tissue without cooling or freezing the tissue, in order to ablate the tissue. Tissue may also be ablated by repeatedly freezing and thawing a volume of tissue, without having to apply microwave energy to it. The electrosurgical apparatus therefore provides a flexible tissue ablation tool, as it enables different ablation techniques to be combined depending on the requirements of a particular situation.

Also disclosed herein is a method for treating biological tissue, the method comprising: non-percutaneously inserting an instrument cord of a surgical scoping device into a patient, the surgical scoping device having an instrument channel running along its length; conveying an electrosurgical instrument as described above along the instrument channel to a treatment zone at a distal end thereof; flowing a tissue-freezing fluid through the fluid feed to freeze biological tissue in the treatment zone; and after biological tissue is frozen in the treatment zone, delivering microwave energy to the radiating tip portion. Any feature of the electrosurgical apparatus and system discussed herein may be utilised in the method. For example, the method may include detecting a temperature in the treatment zone, and controlling delivery of the microwave energy based on the detected temperature. Alternatively or additionally, the method may include detecting an impedance in the treatment zone, and controlling delivery of the microwave energy based on the detected impedance.

Herein, the terms "proximal" and "distal" refer to the ends of a structure (e.g. electrosurgical instrument, coaxial feed cable, etc.) further from and closer to the treatment zone respectively. Thus, in use the proximal end of the structure is accessible by a user, whereas the distal end is closer to the treatment site, i.e. the patient.

The term "conductive" is used herein to mean electrically conductive, unless the context dictates otherwise.

The term "longitudinal" used below refers to the direction along the instrument channel parallel to the axis of the coaxial transmission line. The term "lateral" refers to a direction that is perpendicular to the longitudinal direction. The term "inner" means radially closer to the centre (e.g. axis) of the instrument channel. The term "outer" means radially further from the centre (axis) of the instrument channel.

The term "electrosurgical" is used in relation an instrument, apparatus or tool which is used during surgery and which utilises microwave electromagnetic (EM) energy. Herein, "microwave EM energy" may mean electromagnetic energy having a stable fixed frequency in the range 300 MHz to 100 GHz, preferably in the range 1 GHz to 60 GHz. Preferred spot frequencies for the microwave EM energy include 915 MHz, 2.45 GHz, 5.8 GHz, 14.5 GHz, 24 GHz. 5.8 GHz may be preferred.

In use, the treatment zone may include biological tissue in a patient's lungs, uterus, gastrointestinal tract or other organs.

### BRIEF DESCRIPTION OF THE DRAWINGS

Examples of the invention are discussed below with reference to the accompanying drawings, in which:
Fig. 1 is a schematic diagram of an electrosurgical apparatus for tissue ablation that is an embodiment of the invention;
Fig. 2 is a schematic cross-sectional view of a distal end of an ablation instrument suitable for use in the invention;
Fig. 3 is a schematic cross-sectional view of a distal end of another ablation instrument suitable for use in the invention;
Fig. 4A is a schematic illustration of a tissue ablation method that is an embodiment of the invention;
Fig. 4B is a schematic illustration of another tissue ablation method that is an embodiment of the invention; and
Fig. 5 is a graph of calculated skin effect versus frequency over a range of microwave frequencies used for tissue ablation.

### DETAILED DESCRIPTION; FURTHER OPTIONS AND PREFERENCES

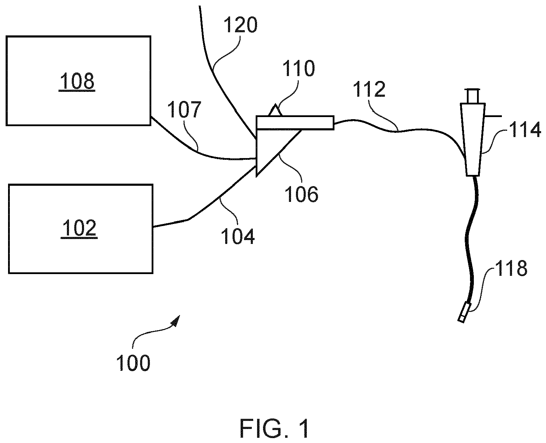

Fig. 1 is a schematic diagram of a complete electrosurgical apparatus 100 that is an embodiment of the invention. The apparatus 100 is arranged to treat biological tissue (e.g. a tumour, lesion or fibroid) using microwave energy delivered from a microwave antenna. The apparatus 100 is capable of freezing a volume of biological tissue, and ablating tissue surrounding the frozen tissue by applying microwave energy to the frozen tissue. Applying microwave energy to frozen tissue enables microwave energy to be transmitted further into a sample of tissue, as frozen tissue does not dissipate microwave energy as strongly as non-frozen tissue. This enables the total volume of tissue which can be treated by the applied microwave energy to be increased, without having to increase the amount of microwave energy delivered.

The system 100 comprises a generator 102 for controllably supplying microwave energy. A suitable generator for this purpose is described in WO 2012/076844. The generator may be arranged to monitor reflected signals received back from the instrument in order to determine an appropriate power level for delivery. For example, the generator may be arranged to calculate an impedance seen at the distal end of the instrument in order to determine an optimal delivery power level.

The generator 102 is connected to an interface joint 106 by an interface cable 104. The interface joint 106 is also connected to a cryogen supply unit 108, such as a cryogen-carrying vessel, via a cryogen conveying conduit 107. If needed, the interface joint 106 can house an instrument control mechanism that is operable by sliding a trigger 110, e.g. to control longitudinal (i.e. back and forth) movement of one or more control wires or push rods (not shown). If there is a plurality of control wires, there may be multiple sliding triggers on the interface joint to provide full control. An exhaust conveying conduit 120 may also be connected to the interface joint 106, through which used cryogen and/or exhaust gas may exit. The function of the interface joint 106 is to combine the inputs from the generator 102, cryogen supply unit 108, the exhaust conveying conduit 120 and instrument control mechanism into a single flexible shaft 112, which extends from the distal end of the interface joint 106.

The flexible shaft 112 is insertable through the entire length of a working (instrument) channel of a surgical scoping device 114, such as a bronchoscope, endoscope, gastroscope, laparoscope or the like. The flexible shaft 112 has a distal assembly 118 (not drawn to scale in Fig. 1) that is shaped to pass through the working channel of the surgical scoping device 114 and protrude (e.g. inside the patient) at the distal end of the surgical scoping device's working channel. The distal end assembly 118 includes a microwave antenna for delivering microwave energy and a tissue-freezing element (not shown) connected to the cryogen conveying conduit 107 for freezing tissue. The tip configuration is discussed in more detail below.

Cryogen may be delivered to the tissue-freezing element from the cryogen supply unit 108 via the cryogen conveying conduit 107. In certain embodiments, the cryogen is used to cool the tissue-freezing element (e.g. via heat exchange processes). In this case, the cryogen may flow into the tissue-freezing element via the cryogen conveying conduit 107, and back out of the tissue-freezing element via the exhaust conveying conduit 120. In other embodiments, the tissue-freezing element includes a nozzle which is used to spray cryogen onto a target area. The cryogen may then escape from the target area via the exhaust conveying conduit 120, to avoid build-up of pressure in the target area. The proximal end of the exhaust conveying conduit 120 may be open to the atmosphere, or it may be connected to a collection chamber (not shown) where used cryogen may be collected. Different cryogen supply units may be connected to the cryogen conveying conduit 107 depending on the cryogen to be used.

The structure of the distal assembly 118 may be arranged to have a maximum outer diameter suitable for passing through the working channel. Typically, the diameter of a working channel in a surgical scoping device such as an endoscope is less than 4.0 mm, e.g. any one of 2.8 mm, 3.2 mm, 3.7 mm, 3.8mm. The length of the flexible shaft 112 can be equal to or greater than 1.2 m, e.g. 2 m or more. In other examples, the distal assembly 118 may be mounted at the distal end of the flexible shaft 112 after the shaft has been inserted through the working channel (and before the instrument cord is introduced into the patient). Alternatively, the flexible shaft 112 can be inserted into the working channel from the distal end before making its proximal connections. In these arrangements, the distal end assembly 118 can be permitted to have dimensions greater than the working channel of the surgical scoping device 114.

The apparatus described above is one way of introducing the device. Other techniques are possible. For example, the device may also be inserted using a catheter.

The invention seeks to provide a device that can ablate biological tissue by applying microwave energy directly to the tissue, and/or by freezing a volume of tissue and applying microwave energy to the frozen tissue. The device is particularly suited to the ablation of tissue in the lungs or uterus, however it may be used to ablate tissue in other organs. In order to efficiently ablate target tissue, the microwave antenna and tissue-freezing element should be located as close as possible (and in many cases inside) the target tissue. In order to reach the target tissue (e.g. in the lungs), the device will need to be guided through passageways (e.g. airways) and around obstacles. This means that the instrument will ideally be flexible and have a small cross section. Particularly, the device should be very flexible near its tip, where it may need to be steered along passageways such as bronchioles which can be narrow and winding.

It is also preferable that the device can be operated alongside other instruments to enable practitioners to receive information from the target site. For example, an endoscope may aid the steering of the instruments around obstacles and to a desired position. Other instruments may include a thermometer or camera.

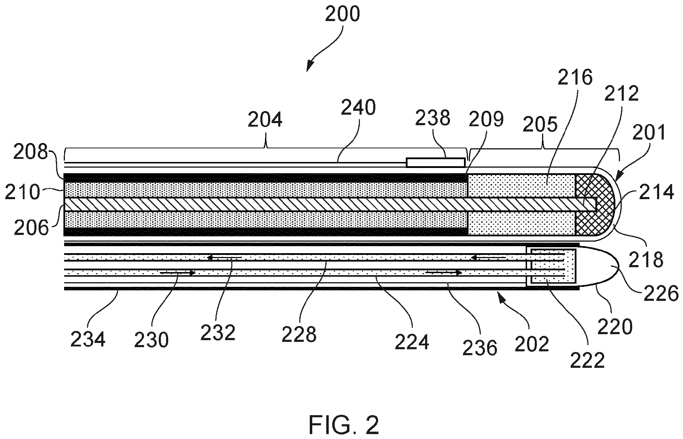

Fig. 2 is a schematic cross-sectional view of a distal end of an electrosurgical device 200 that is an embodiment of the invention. The electrosurgical device 200 includes an electrosurgical instrument 201 and a cryogenic instrument 202.

Electrosurgical instrument 201 includes a coaxial feed cable 204 that is connected at its proximal end to a generator (such as generator 102) in order to convey microwave energy. The coaxial feed cable 204 comprises an inner conductor 206, which is separated from an outer conductor 208 by a first dielectric material 210. The coaxial feed cable 204 is preferably low loss for microwave energy. A choke (not shown) may be provided on the coaxial feed cable 204 to inhibit back propagation of microwave energy reflected from the distal end and therefore limit backward heating along the device.

The coaxial feed cable 204 terminates at its distal end with a radiating tip portion 205 for radiating microwave energy. In this embodiment, the radiating tip portion 205 comprises a distal conductive section 212 of the inner conductor 206 that extends before a distal end 209 of the outer conductor 208. The distal conductive section 212 is surrounded at its distal end by a dielectric tip 214 formed from a second dielectric material, which is different from the first dielectric material 210. The length of the dielectric tip 214 is shorter than the length of the distal conductive section 212. An intermediate dielectric sleeve 216 surrounds the distal conductive section 212 between the distal end of the coaxial cable 202 and the proximal end of the dielectric tip 214. The intermediate dielectric sleeve 216 is formed from a third dielectric material, which is different from the second dielectric material but which may be the same as the first dielectric material 210. The dielectric tip 214 may have any suitable distal shape. In Fig. 2 it has a dome shape, but this is not necessarily essential. For example, it may be cylindrical, conical, etc. However, a smooth dome shape may be preferred because it increases the mobility of the antenna as it is manoeuvred through small channels. The electrosurgical instrument 201 is housed in a protective sheath 218 which electrically insulates the electrosurgical instrument 201. The protective sheath 218 may be made of, or coated with, a non-stick material such as PTFE to prevent tissue from sticking to the instrument.

The properties of the intermediate dielectric sleeve 216 are preferably chosen (e.g. through simulation or the like) so that the radiating tip portion 205 forms a quarter wave impedance transformer for matching the input impedance of the generator into a biological tissue load in contact with the radiating tip portion 205. This configuration of the radiating tip portion 205 may produce an approximately spherical radiation pattern about the radiating tip portion 205. This enables the user to accurately radiate target tissue and reduces radiation of or damage to healthy tissue. Depending on the radiation pattern required, different radiating tip portion configurations may be used. For example, an asymmetric radiation pattern can be produced by extending the outer conductor 208 along one side of the radiating tip portion 205.

The cryogenic instrument 202 includes a tissue-freezing element 220 at a distal end of the cryogenic instrument 202, located near the radiating tip portion 205. The tissue-freezing element 220 includes a reservoir 222 for receiving cryogen delivered by a cryogen conveying conduit 224. The tissue-freezing element 220 also includes a tip portion 226 which is thermally linked to the reservoir 222 so that the tip portion 226 may be cooled by cryogen in the reservoir 222. The tissue-freezing element 220 may for example be formed of a single piece of thermally conductive material, with the reservoir 222 being formed by a cavity in the material.

The cryogenic instrument 202 also includes an exhaust conveying conduit 228 connected to the reservoir 222 for conveying cryogen from the reservoir 222 to a proximal end of the apparatus where the cryogen may be collected or disposed of. Thus, as indicated by flow direction arrows 230, cryogen may be conveyed through the cryogen conveying conduit 224 so that it is delivered into the reservoir 222. The cryogen can accumulate in the reservoir 222, causing the tip portion 226 of the tissue-freezing element 220 to cool down, so that it may be used to freeze tissue. Excess cryogen in the reservoir 222 may be evacuated through the exhaust conveying tube 228 as indicated by arrows 232, to avoid a build-up of pressure in the reservoir 222. In the case of a liquid cryogen (e.g. liquid nitrogen), the cryogen may expand into a gas as it absorbs heat from the tip portion. The gas may also form part of the cryogen which is evacuated through the exhaust conveying conduit 228. Both the cryogen conveying conduit 224 and the exhaust conveying conduit 228 may be fitted with one way valves to ensure that cryogen flows only in the direction indicated by arrows 230 and 232. A pump located at the proximal end of the electrosurgical device may be used to circulate cryogen in the cryogen conveying conduit 224 and the exhaust conveying conduit 228.

A thermally insulating sleeve 234 surrounds the cryogen conveying conduit 224, the exhaust conveying conduit 228 and part of the tissue-freezing element 220. The thermally insulating sleeve 234 prevents heat from being exchanged between cryogen in the conduits and the surrounding environment. Additionally or alternatively, the cryogen conveying conduit 224 and the exhaust conveying conduit 228 may themselves be made of a thermally insulating material. Thermal insulation of the conduits may be improved by creating a vacuum in the space inside the thermally insulating sleeve 234. Thermal insulation of the conduits ensures that only tissue in the vicinity of the tissue-freezing element 220 may be frozen, thus avoiding accidental cold damage to the patient.

The tissue-freezing element 220 may be slidable within the thermally insulating sleeve 234 along its length. The fit between the outer surface of the tissue-freezing element 220 and the inner surface of the thermally insulating sleeve may be sufficiently tight so that it forms an air-tight sliding seal. The reservoir 222 may be slidably connected to conduits 224 and 228 (e.g. via sliding seals) to enable the tissue-freezing element 220 to move relative to the conduits 224 and 228. Alternatively, the connections between the reservoir 222 and the conduits 222 and 228 may be fixed, such that the conduits 224 and 228 move with the tissue-freezing element in the thermally insulating sleeve 234. The tissue-freezing element 220 can be slid along the thermally insulating sleeve 234 using a control wire 236 which passes through the thermally insulating sleeve 234 and is connected at one end to the tissue-freezing element 220. The tissue-freezing element may be fully or partially retracted into the thermally insulating sleeve 234, so that its tip portion 226 does not protrude beyond the distal end of the electrosurgical instrument 201. When a user wishes to use the tissue-freezing element 220 to freeze biological tissue, the tissue-freezing element 220 may be exposed such that it protrudes beyond the distal end of the electrosurgical instrument 201, so that it may come into contact with target tissue. The tissue-freezing element 220 may be placed in its retracted position when the instrument is being navigated to a target area, to avoid the tip portion 226 catching on tissue or causing accidental injury. Alternative mechanisms to that described above are possible for enabling the tissue-freezing element 220 to move relative to the electrosurgical instrument 201.

A heater and temperature sensor (not shown) may be mounted near the tip portion 226 of the tissue-freezing element 220 to enable accurate control of the temperature at the tip portion 226. The heater may be a resistive chip which heats up when an electrical current is passed through it. By balancing heat generated by the heater with the cooling power provided by the cryogen, a stable temperature can be obtained at the tip portion 226. A PID controller may be used to control the temperature to the tip portion 226. The heater may also be used to heat the tip portion 226 in order to thaw frozen tissue.

The cryogenic instrument 202 may be fixed relative to the electrosurgical instrument 201, so that the two components form a single integrated device which is configured to fit in the working channel of an endoscope. For example, the thermally insulating sleeve 234 may be secured to the protective sheath 218 of the electrosurgical instrument 201.

The tip portion 226 of the tissue-freezing element 220 shown in Fig. 2 is dome shaped. However, other shapes are possible. For example, it may be cylindrical, conical, etc. In general, it is desirable for the shape of the tip portion 226 to be such that it maximises heat transfer between the tip portion 226 and target tissue, in order to efficiently freeze the tissue. Therefore it may be desirable to use a shape which maximises contact area between the tip portion 226 and the target tissue. In some cases, the tip portion 226 may have a sharp tip so that it can pierce tissue and be inserted inside target tissue. In some examples the tip portion 226 and dielectric tip 214 may be or form part of a common tip structure for the device 200.

The distal end of the electrosurgical device 200 may also include a sensor 238 located near the radiating tip portion 205, for measuring a property of tissue in a treatment zone around the radiating tip portion. Measurements can be obtained from the sensor 238 via wiring 240. For example, the sensor 238 may be a temperature sensor for measuring a temperature of tissue in the treatment zone. The sensor 238 may also be a pressure sensor, for measuring a change in pressure in the treatment zone. Measurements from the sensor 238 may be used to determine when tissue in the treatment zone is frozen, in order to determine when to apply microwave energy to the treatment zone.

In some embodiments, the electrosurgical device 200 may also include an outer sheath in which the components at the distal end of the device are housed. The outer sheath may have an aperture through which the tissue-freezing element 220 may protrude. The outer sheath may have a smooth shape so that no sharp corners are presented to biological tissue, in order to avoid accidental injuries.

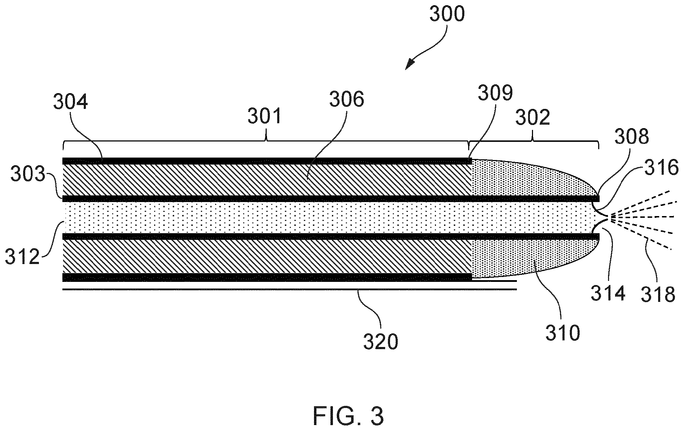

Fig. 3 is a schematic cross-sectional view of a distal end of an electrosurgical device 300 that is another embodiment of the invention. In this embodiment, the cryogenic instrument is integrated into the electrosurgical instrument. The electrosurgical device 300 includes a coaxial feed cable 301, which can be connected at its proximal end to a generator (e.g. generator 102) in order to convey microwave energy. The coaxial feed cable 301 comprises an inner conductor 303, which is separated from an outer conductor 304 by a first dielectric material 306. The coaxial feed cable 301 is preferably low loss for microwave energy. A choke (not shown) may be provided on the coaxial cable to inhibit back propagation of microwave energy reflected from the distal end and therefore limit backward heating along the device.

The coaxial feed cable 301 terminates at its distal end with a radiating tip portion 302 for radiating microwave energy. In this embodiment, the radiating tip portion 302 comprises a distal conductive section 308 of the inner conductor 303 that extends before a distal end 309 of the outer conductor 304. The inner conductor 303 is hollow, with an inner surface of the inner conductor defining a channel 312 running through the inner conductor 303. The distal conductive section 308 is surrounded at its distal end by a dielectric tip 310 formed from a second dielectric material, which is different from the first dielectric material 306. The dielectric tip 310 is dome-shaped and has a channel running through it, and through which the inner conductor 303 passes. An aperture 314 is formed at the distal end of the inner conductor 303.

The channel 312 may be connected at a proximal end to a cryogen supply unit (e.g. cryogen supply unit 108), so that channel 312 may act as a cryogen conveying conduit of a cryogenic instrument. A nozzle 316 which is fluidly connected to the channel 312 is located near the aperture 314 of the inner conductor. The nozzle 316 is arranged to spray cryogen conveyed through the channel 312 towards a target site in front of the radiating tip portion 302 (i.e. to the right in Fig. 3), as illustrated by dashed lines 318. The nozzle 316 may for example be a slit valve, however other types of nozzle are also possible. The nozzle 316 is configured to prevent fluid from the target site from entering the channel 312, and so may include a one-way valve. In some embodiments, the nozzle 316 may include a fine tube in order to provide a concentrated and directed cryogen spray. In some embodiments, the nozzle 316 may be slidable in the channel 312 (e.g. using one or more control wires), so that the nozzle can be made to protrude beyond the radiating tip portion 302. This way the nozzle 316 may be retracted when the electrosurgical device 200 is being guided into position, and then it may be deployed to spray target tissue.

The electrosurgical device 200 further includes a decompression tube 320, through which gas in a treatment zone surrounding the radiating tip portion 302 may escape. This avoids pressure build-up in the treatment zone, which could lead to internal damage to the patient. In the case where the cryogen is a cold gas, cold gas sprayed from the nozzle 316 may cool tissue in the treatment zone and then exit the treatment zone via the decompression tube 320. In the case where the cryogen is a cryogenic liquid, cryogenic liquid sprayed from the nozzle 316 may cool tissue in the treatment zone and expand into a gas. The resulting gas may then exit the treatment zone via the decompression tube 320.

Preferably the decompression tube 320 is configured so that gas may only flow from the distal end of the electrosurgical device 200 (i.e. from the treatment zone) to the proximal end of the electrosurgical device 200. This is to avoid gas entering the treatment zone via the decompression tube 320. The decompression tube 320 may vent to atmosphere at its proximal end, or it may be connected to a gas collection chamber. The decompression tube 320 may also be fitted with a pressure relief valve, which is configured to allow gas to flow along the decompression tube 320 when pressure in the treatment zone reaches a predetermined threshold. In this manner, the pressure in the treatment zone may be maintained at a safe level.

The first dielectric material 306 may be a thermally insulating material, or may include a thermally insulating layer, so that the outer conductor 304 is not cooled by cryogen in the channel 312. In this manner the outer surface of the coaxial feed cable 301 will not become cold when cryogen is run through the channel 312, thus avoiding the risk of freezing parts of the patient outside the treatment zone. Alternatively, the coaxial feed cable 301 may include a thermally insulating sleeve and/or a vacuum jacket around the outer surface of the outer conductor 304.

Cryogen running through the channel 312 may cool the inner conductor 303 and dissipate heat generated by any microwave energy propagated through the coaxial feed cable 301. This enables the amount of microwave energy carried by the coaxial feed cable 301 to be increased without overheating the coaxial feed cable 301. The configuration shown in Fig. 3 therefore enables larger amounts of microwave energy to be applied, which may increase the volume of tissue ablated by the microwave energy.

The electrosurgical device 300 may also include one or more sensors and/or a heater, similar to those discussed in relation to Fig. 2.

It should be noted that different combinations of features from the embodiments shown in Figs. 2 and 3 are possible, with the embodiments shown in Figs 2 and 3 being given merely by way of example. For example, the cryogenic instrument 202 of Fig. 2 may be inserted through a hollow inner conductor of an electrosurgical instrument similar to that shown in Fig. 3. In this manner, the cryogen conveying conduit 224, exhaust conveying conduit 228 and tissue-freezing element 220 may be contained within a channel similar to channel 312, such that the cryogenic instrument 202 is fully integrated into the electrosurgical instrument. In another example, the cryogenic system 202 of Fig. 2 may be replaced by a cryogen-spraying mechanism including a nozzle and a decompression tube similar to those described in relation to Fig. 3.

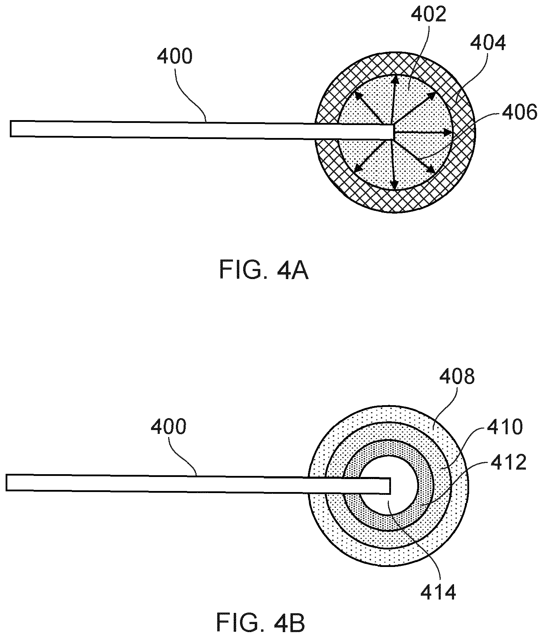

Fig. 4A shows a schematic illustration of biological tissue ablation using an electrosurgical device according to the invention. The distal end of an electrosurgical device 400, such as those described in relation to Figs. 2 and 3, is inserted into target tissue which is to be ablated. Using the cryogenic instrument of the electrosurgical device 400, a volume of tissue 402 around the distal end of the electrosurgical device 400 is frozen. This is done by flowing a cryogen through the cryogen conveying conduit to a tissue-freezing element at the distal end of the electrosurgical device 400. Where the electrosurgical device in Fig. 2 is used, the reservoir 222 may be filled with cryogen to cool the tip portion 226 of the tissue-freezing element 220. The tissue-freezing element 220 may then be slid out of the thermally insulating sleeve 234 so that it comes into contact with the target tissue and causes the volume of tissue 402 to freeze. Where the electrosurgical device in Fig. 3 is used, cryogen from the cryogen conveying conduit may be sprayed onto the target tissue to freeze the volume of tissue 402. The volume 402 of tissue which is frozen may depend on the flow rate of cryogen through the cryogen conveying conduit and/or the temperature of the tissue-freezing element (which in some embodiments may be controlled using a heater).

Once the volume of tissue 402 is frozen, microwave energy is delivered to the radiating tip portion of the electrosurgical instrument so that microwave energy is applied to the frozen tissue. The microwave energy is transmitted with relatively low loss through the volume 402 of frozen tissue and into a surrounding layer of non-frozen tissue 404, as indicated by arrows 406. The microwave energy rapidly dissipates as heat in the layer of tissue 404, causing ablation of the layer of tissue 404. While the layer of tissue 404 is being ablated, the volume of tissue 402 may be kept frozen, e.g. by maintaining a constant flow of cryogen through the cryogen conveying conduit and/or controlling the temperature of the tissue-freezing element.

The state of the tissue (e.g. frozen or non-frozen) surrounding the distal end of the electrosurgical device 400 may be determined by measuring various properties of the tissue. For example, using one or more sensors mounted near the distal end of the electrosurgical device 400, temperature and/or pressure can be measured to give an indication of whether the tissue is frozen. The electrosurgical instrument may be used to measure the impedance of the tissue surrounding its distal end, and thus determine whether the tissue is frozen. The impedance measurement may also be used to estimate the volume of tissue around the distal end which is frozen. Impedance of the tissue may be measured by sending a pulse of microwave energy down the coaxial feed cable to the radiating tip portion and measuring any microwave energy reflected back up the coaxial feed cable.

Once the tissue in layer 404 has been ablated, the tissue in volume 402 may be gradually allowed to thaw so that it can be progressively ablated by applied microwave energy. The tissue can be allowed to thaw by reducing the flow of cryogen through the cryogen conveying conduit and/or increasing the temperature of the tissue-freezing element (e.g. using a heater mounted thereon). By appropriately controlling the flow of cryogen through the cryogen conveying conduit and/or the temperature of the tissue-freezing element, the volume of tissue which is frozen may be reduced in stages. At each stage, microwave energy may be applied to ablate a layer of tissue surrounding the frozen tissue which was previously frozen.

This process is illustrated in Fig. 4B. Initially, tissue layers 414, 412 and 410 are frozen, so that outer layer 408 may be ablated when microwave energy is applied to the frozen tissue. Then, layer 410 is allowed to thaw, keeping layers 414 and 412 frozen, such that layer 410 may be ablated by microwave energy. Layer 412 is then thawed, so that it may also be ablated. Finally, the innermost layer 414 is thawed and ablated by direct application of microwave energy. In this manner, the total volume of tissue which can be ablated is defined by an outer surface of the layer of tissue 408. This volume may be much greater than the volume of tissue which can be ablated by applying the same amount of microwave energy directly to non-frozen tissue.

A controller may be used to control the various steps in the ablation process. The controller may be configured to obtain measurements from one or more sensors located near the distal end of the electrosurgical instrument in order to determine whether tissue in a treatment zone around the radiating tip portion of the electrosurgical instrument is frozen. The controller may also be configured to carry out impedance measurements to determine if the tissue in the treatment zone is frozen. Depending on the result of the determination, the controller may be configured to adjust the flow of cryogen in the cryogen conveying conduit and/or the temperature of the tissue-freezing element (e.g. to increase or decrease the volume of frozen tissue in the treatment zone). Once it is determined that a desired volume of tissue has been frozen, the controller may be configured to deliver microwave energy to the radiating tip portion. The controller may also be configured to successively ablate tissue layers as described in relation to Fig. 4B.

The controller may be a conventional computing device having software installed thereon for carrying out the various steps described above. The computer may be connected to the generator 102, so that it can control the supply of microwave energy to the radiating tip portion of the electrosurgical instrument. The computer may also be connected to the cryogen supply unit 108 to control the flow of cryogen through the cryogen conveying conduit (e.g. by controlling a valve in the cryogen supply unit 108). Outputs from any sensors on the electrosurgical device may be connected to the controller, so that it can obtain measurements from the sensors. If a heater is disposed on the electrosurgical apparatus, its input may also be connected to the controller. In this manner the controller provides an automated system for performing tissue ablation.

## Claims

1. An electrosurgical apparatus (100) for treating biological tissue, the apparatus comprising:
an electrosurgical generator (102) arranged to supply microwave electromagnetic (EM) energy;
a tissue-freezing fluid supply (108);
an electrosurgical instrument (201) connected to receiving the microwave EM energy from the electrosurgical generator (102) and to receive the tissue-freezing fluid from the tissue-freezing fluid supply (108); and
a controller configured to:
cause the tissue-freezing fluid to flow through a fluid feed to a thermal transfer portion to freeze biological tissue in the treatment zone;
detect conditions in the treatment zone to determine if biological tissue in the treatment zone is frozen; and
in response to determining that biological tissue in the treatment zone is frozen, cause the microwave energy to be delivered from the radiating tip,
wherein the electrosurgical instrument (201) for treating biological tissue comprises:
a transmission line for conveying microwave electromagnetic (EM) energy;
a radiating tip (205) mounted at a distal end of the transmission line to receive and radiate the microwave EM energy from the transmission line into a treatment zone around the radiating tip;
the fluid feed for conveying a tissue-freezing fluid to the treatment zone; and
the thermal transfer portion connected to receive the tissue-freezing fluid from the fluid feed at a distal end of the transmission line,
wherein the thermal transfer portion is arranged to provide thermal communication between the tissue-freezing fluid and biological tissue in the treatment zone to freeze the biological tissue in the treatment zone, and
wherein the thermal transfer portion includes a tissue-freezing element (220) that is movable between an exposed position where it protrudes distally beyond the radiating tip, and a retracted position in which it is set back from the radiating tip.

2. An electrosurgical apparatus (100) according to claim 1, wherein the tissue-freezing fluid is a cryogenic liquid or gas.

3. An electrosurgical apparatus (100) according to any preceding claim, wherein the fluid feed is arranged to circulate the tissue-freezing fluid through the thermal transfer portion.

4. An electrosurgical apparatus (100) according to any preceding claim, wherein the fluid feed comprises a delivery conduit for conveying the tissue-freezing fluid to the thermal transfer portion, and an exhaust conduit (228) for conveying the tissue-freezing fluid away from the thermal transfer portion.

5. An electrosurgical apparatus (100) according to any preceding claim, wherein the thermal transfer portion comprises an enclosed reservoir (222) for receiving the tissue-freezing fluid.

6. An electrosurgical apparatus (100) according to any one of claims 1 to 4, wherein the thermal transfer portion includes an outlet (314) for delivering the tissue-freezing fluid into the treatment zone.

7. An electrosurgical apparatus (100) according to claim 6, wherein the outlet includes a nozzle (316), the nozzle (316) being arranged to spray the tissue-freezing fluid into the treatment zone.

8. An electrosurgical apparatus (100) according to any preceding claim, wherein the transmission line and the fluid feed are within a common cable.

9. An electrosurgical apparatus (100) according to any preceding claim, wherein the fluid feed is integrated with the transmission line.

10. An electrosurgical apparatus (100) according to claim 9, wherein the transmission line is a coaxial transmission line (204) comprising an inner conductor (206), an outer conductor (208), and a dielectric material (210) separating the inner conductor (206) and the outer conductor (208), and wherein the inner conductor (206) is hollow to provide a passageway for the fluid feed.

11. An electrosurgical apparatus (100) according to any preceding claim including a temperature sensor (238) mounted at a distal end of the transmission line to detect a temperature of the treatment zone.

12. An electrosurgical apparatus (100) according to any preceding claim, wherein the thermal transfer portion further comprises a heating element.

13. An electrosurgical apparatus (100) according to any preceding claim, wherein the controller is configured to, in response to determining that biological tissue in the treatment zone is frozen, reduce or stop the flow of tissue-freezing fluid through the fluid feed.

14. An electrosurgical apparatus (100) according to any preceding claim, wherein the controller is configured to determine whether the biological tissue in the treatment zone is frozen based on any one or more of:
a detected impedance of the treatment zone, and
a detected temperature of the treatment zone.

15. An electrosurgical apparatus (100) according to any preceding claim, further comprising:
a surgical scoping device (114) having a flexible insertion cord for non-percutaneous insertion into a patient's body,
wherein the flexible insertion cord has an instrument channel running along its length, and
wherein the electrosurgical instrument (201) is dimensioned to fit within the instrument channel.

## Patentansprüche

1. Elektrochirurgisches Gerät (100) zur Behandlung von biologischem Gewebe, wobei das Gerät Folgendes umfasst:
einen elektrochirurgischen Generator (102), der angeordnet ist, um elektromagnetische (EM) Mikrowellenenergie bereitzustellen;
eine Gewebeeinfrierfluidversorgung (108);
ein elektrochirurgisches Instrument (201), das verbunden ist, um die EM-Mikrowellenergie vom elektrochirurgischen Generator (102) zu empfangen und das Gewebeeinfrierfluid von der Gewebeeinfrierfluidversorgung (108) zu empfangen; und
eine Steuerung, die ausgelegt ist, um:
zu bewirken, dass das Gewebeeinfrierfluid durch eine Fluidzuleitung zu einem Wärmeübertragungsabschnitt fließt, um biologisches Gewebe in der Behandlungszone einzufrieren;
Bedingungen in der Behandlungszone zu detektieren, um zu bestimmen, ob biologisches Gewebe in der Behandlungszone gefroren ist; und
als Reaktion auf die Bestimmung, dass biologisches Gewebe in der Behandlungszone gefroren ist, zu bewirken, dass die Mikrowellenenergie von der Strahlungsspitze abgegeben wird,
wobei das elektrochirurgische Instrument (201) zur Behandlung von biologischem Gewebe Folgendes umfasst:
eine Übertragungsleitung zum Transport von elektromagnetischer (EM) Mikrowellenenergie;
eine Strahlungsspitze (205), die an einem distalen Ende der Übertragungsleitung angebracht ist, um die EM-Mikrowellenenergie von der Übertragungsleitung zu empfangen und in eine Behandlungszone um die Strahlungsspitze abzustrahlen;
die Fluidzuleitung zum Transport eines Gewebeeinfrierfluids zur Behandlungszone; und
den Wärmeübertragungsabschnitt, der verbunden ist, um das Gewebeeinfrierfluid von der Fluidzuleitung an einem distalen Ende der Übertragungsleitung zu empfangen,
wobei der Wärmeübertragungsabschnitt angeordnet ist, um thermische Kommunikation zwischen dem Gewebeeinfrierfluid und biologischem Gewebe in der Behandlungszone bereitzustellen, um das biologische Gewebe in der Behandlungszone einzufrieren, und
wobei der Wärmeübertragungsabschnitt ein Gewebeeinfrierelement (220) umfasst, das zwischen einer exponierten Stellung, in der es distal über die Strahlungsspitze vorsteht, und einer eingefahrenen Stellung, in der es hinter der Strahlungsspitze liegt, bewegbar ist.

2. Elektrochirurgisches Gerät (100) nach Anspruch 1, wobei das Gewebeeinfrierfluid eine kryogene Flüssigkeit oder ein kryogenes Gas ist.

3. Elektrochirurgisches Gerät (100) nach einem der vorangegangenen Ansprüche, wobei die Fluidzuleitung angeordnet ist, um das Gewebeeinfrierfluid durch den Wärmeübertragungsabschnitt zu zirkulieren.

4. Elektrochirurgisches Gerät (100) nach einem der vorangegangenen Ansprüche, wobei die Fluidzuleitung eine Zufuhrleitung zum Transport des Gewebeeinfrierfluids zum Wärmeübertragungsabschnitt und eine Ableitung (228) zum Transport des Gewebeeinfrierfluids weg vom Wärmeübertragungsabschnitt umfasst.

5. Elektrochirurgisches Gerät (100) nach einem der vorangegangenen Ansprüche, wobei der Wärmeübertragungsabschnitt einen abgeschlossenen Behälter (222) zur Aufnahme des Gewebeeinfrierfluids umfasst.

6. Elektrochirurgisches Gerät (100) nach einem der Ansprüche 1 bis 4, wobei der Wärmeübertragungsabschnitt einen Auslass (314) zur Zufuhr des Gewebeeinfrierfluids in die Behandlungszone umfasst.

7. Elektrochirurgisches Gerät (100) nach Anspruch 6, wobei der Auslass eine Düse (316) umfasst, wobei die Düse (316) angeordnet ist, um das Gewebeeinfrierfluid in die Behandlungszone zu sprühen.

8. Elektrochirurgisches Gerät (100) nach einem der vorangegangenen Ansprüche, wobei die Übertragungsleitung und die Fluidzuleitung in einem gemeinsamen Kabel sind.

9. Elektrochirurgisches Gerät (100) nach einem der vorangegangenen Ansprüche, wobei die Fluidzuleitung mit der Übertragungsleitung eingebunden ist.

10. Elektrochirurgisches Gerät (100) nach Anspruch 9, wobei die Übertragungsleitung eine koaxiale Übertragungsleitung (204) ist, die einen inneren Leiter (206), einen äußeren Leiter (208) und ein dielektrisches Material (210), das den inneren Leiter (206) vom äußeren Leiter (208) trennt, umfasst und wobei der innere Leiter (206) hohl ist, um einen Durchlass für die Fluidzuleitung bereitzustellen.

11. Elektrochirurgisches Gerät (100) nach einem der vorangegangenen Ansprüche, das einen Temperatursensor (238) umfasst, der an einem distalen Ende der Übertragungsleitung angebracht ist, um eine Temperatur der Behandlungszone zu detektieren.

12. Elektrochirurgisches Gerät (100) nach einem der vorangegangenen Ansprüche, wobei der Wärmeübertragungsabschnitt weiters ein Heizelement umfasst.

13. Elektrochirurgisches Gerät (100) nach einem der vorangegangenen Ansprüche, wobei die Steuerung ausgelegt ist, um als Reaktion auf die Bestimmung, dass biologisches Gewebe in der Behandlungszone eingefroren ist, den Fluss von Gewebeeinfrierfluid durch die Fluidzuleitung zu verringern oder zu stoppen.

14. Elektrochirurgisches Gerät (100) nach einem der vorangegangenen Ansprüche, wobei die Steuerung ausgelegt ist, um basierend auf einem oder mehreren der Folgenden zu bestimmen, ob biologisches Gewebe in der Behandlungszone eingefroren ist:
eine detektierte Impedanz der Behandlungszone und
eine detektierte Temperatur der Behandlungszone.

15. Elektrochirurgisches Gerät (100) nach einem der vorangegangenen Ansprüche, das weiters Folgendes umfasst:
eine chirurgische Sondiervorrichtung (114) mit einem flexiblen Schlauch zur nichtperkutanen Einführung in den Körper eines Patienten,
wobei ein Instrumentenkanal der Länge nach durch den flexiblen Einführungsschlauch verläuft und
wobei das elektrochirurgische Instrument (201) so bemessen ist, dass es in den Instrumentenkanal passt.

## Revendications

1. Appareil électrochirurgical (100) pour traiter un tissu biologique, l'appareil, comprenant :
un générateur électrochirurgical (102) agencé pour fournir de l'énergie électromagnétique (EM) de micro-ondes ;
une alimentation en fluide de congélation de tissu (108) ;
un instrument électrochirurgical (201) connecté pour recevoir l'énergie EM de micro-ondes à partir du générateur électrochirurgical (102) et pour recevoir le fluide de congélation de tissu à partir de l'alimentation en fluide de congélation de tissu (108) ; et
un dispositif de commande configuré pour :
amener le fluide de congélation de tissu à s'écouler à travers une alimentation en fluide vers une partie de transfert thermique pour congeler le tissu biologique dans la zone de traitement ;
détecter des conditions dans la zone de traitement afin de déterminer si le tissu biologique dans la zone de traitement est congelé ; et
en réponse à la détermination que le tissu biologique dans la zone de traitement est congelé, amener l'énergie de micro-ondes à être distribuée à partir de la pointe rayonnante,
dans lequel l'instrument électrochirurgical (201) pour traiter un tissu biologique comprend :
une ligne de transmission pour acheminer de l'énergie électromagnétique (EM) de micro-ondes ;
une pointe rayonnante (205) montée au niveau d'une extrémité distale de la ligne de transmission pour recevoir et rayonner l'énergie EM de micro-ondes à partir de la ligne de transmission jusque dans une zone de traitement autour de la pointe rayonnante ;
l'alimentation en fluide pour acheminer un fluide de congélation de tissu vers la zone de traitement ; et
la partie de transfert thermique connectée pour recevoir le fluide de congélation de tissu à partir de l'alimentation en fluide au niveau d'une extrémité distale de la ligne de transmission,
dans lequel la partie de transfert thermique est agencée pour assurer une communication thermique entre le fluide de congélation de tissu et le tissu biologique dans la zone de traitement afin de congeler le tissu biologique dans la zone de traitement, et
dans lequel la partie de transfert thermique inclut un élément de congélation de tissu qui est mobile entre une position exposée dans laquelle il fait saillie distalement au-delà de la pointe rayonnante, et une position rétractée dans laquelle il est en retrait par rapport à la pointe rayonnante.

2. Appareil électrochirurgical (100) selon la revendication 1, dans lequel le fluide de congélation de tissu est un liquide ou un gaz cryogénique.

3. Appareil électrochirurgical (100) selon l'une quelconque des revendications précédentes, dans lequel l'alimentation en fluide est agencée pour faire circuler le fluide de congélation de tissu à travers la partie de transfert thermique.

4. Appareil électrochirurgical (100) selon l'une quelconque des revendications précédentes, dans lequel l'alimentation en fluide comprend un conduit de distribution pour transporter le fluide de congélation de tissu vers la partie de transfert thermique, et un conduit d'évacuation (228) pour transporter le fluide de congélation de tissu loin de la partie de transfert thermique.

5. Appareil électro-chirurgical (100) selon l'une quelconque des revendications précédentes, dans lequel la partie de transfert thermique comprend un réservoir fermé (222) pour recevoir le fluide de congélation de tissu.

6. Appareil électrochirurgical (100) selon l'une quelconque des revendications 1 à 4, dans lequel la partie de transfert thermique inclut une sortie (314) pour distribuer le fluide de congélation de tissu dans la zone de traitement.

7. Appareil électrochirurgical (100) selon la revendication 6, dans lequel la sortie inclut une buse (316), la buse (316) étant agencée pour pulvériser le fluide de congélation de tissu dans la zone de traitement.

8. Appareil électrochirurgical (100) selon l'une quelconque des revendications précédentes, dans lequel la ligne de transmission et l'alimentation en fluide sont à l'intérieur d'un câble commun.

9. Appareil électrochirurgical (100) selon l'une quelconque des revendications précédentes, dans lequel l'alimentation en fluide est intégrée à la ligne de transmission.

10. Appareil électrochirurgical (100) selon la revendication 9, dans lequel la ligne de transmission est une ligne de transmission coaxiale (204) comprenant un conducteur interne (206), un conducteur externe (208), et un matériau diélectrique (210) séparant le conducteur interne (206) et le conducteur externe (208), et dans lequel le conducteur interne (206) est creux pour fournir un passage pour l'alimentation en fluide.

11. Appareil électrochirurgical (100) selon l'une quelconque des revendications précédentes, incluant un capteur de température (238) monté au niveau d'une extrémité distale de la ligne de transmission pour détecter une température de la zone de traitement.

12. Appareil électrochirurgical (100) selon l'une quelconque des revendications précédentes, dans lequel la partie de transfert thermique comprend en outre un élément chauffant.

13. Appareil électrochirurgical (100) selon l'une quelconque des revendications précédentes, dans lequel le dispositif de commande est configuré pour, en réponse à la détermination que du tissu biologique dans la zone de traitement est congelé, réduire ou arrêter l'écoulement de fluide de congélation de tissu à travers l'alimentation en fluide.

14. Appareil électrochirurgical (100) selon l'une quelconque des revendications précédentes, dans lequel le dispositif de commande est configuré pour déterminer si le tissu biologique dans la zone de traitement est congelé sur la base de l'un quelconque ou de plusieurs des éléments suivants :
une impédance détectée de la zone de traitement, et
une température détectée de la zone de traitement.

15. Appareil électrochirurgical (100) selon l'une quelconque des revendications précédentes, comprenant en outre :
un dispositif d'exploration chirurgicale (114) présentant un cordon d'insertion flexible pour une insertion non percutanée dans le corps d'un patient,
dans lequel le cordon d'insertion flexible présente un canal d'instrument sur toute sa longueur, et
dans lequel l'instrument électrochirurgical (201) est dimensionné pour s'adapter à l'intérieur du canal d'instrument.
